(19) Europäisches Patentamt
European Patent Office
Office européen des brevets

(11) Veröffentlichungsnummer: **0 164 669**
**B1**

(12) **EUROPÄISCHE PATENTSCHRIFT**

(45) Veröffentlichungstag der Patentschrift: **23.01.91**

(51) Int. Cl.⁵: **A 61 K 9/32**

(21) Anmeldenummer: **85106791.8**

(22) Anmeldetag: **01.06.85**

(54) **Verfahren zum Überziehen von Arzneiformen.**

(30) Priorität: **13.06.84 DE 3421860**
**19.07.84 DE 3426587**

(43) Veröffentlichungstag der Anmeldung:
**18.12.85 Patentblatt 85/51**

(45) Bekanntmachung des Hinweises auf die Patenterteilung:
**23.01.91 Patentblatt 91/04**

(84) Benannte Vertragsstaaten:
**AT BE CH DE FR GB IT LI NL SE**

(56) Entgegenhaltungen:
**EP-A-0 058 765**
**DE-A-3 049 179**
**DE-C-1 090 381**
**US-A-3 321 451**

**"Wasserlösliche Acrylharze", ROEHM & HAAS GmbH, Darmstadt (1965), pp. 7, 8, 21, 22**

(73) Patentinhaber: **Röhm GmbH**
**Kirschenallee Postfach 4242**
**D-6100 Darmstadt 1 (DE)**

(72) Erfinder: **Lehmann, Klaus, Dr.**
**Schillerstr. 85**
**D-6101 Rossdorf 1 (DE)**
Erfinder: **Dreher, Dieter**
**Hermannstädterweg 34**
**D-6100 Darmstadt (DE)**
Erfinder: **Götz, Harry**
**Gernsheimerstr. 97**
**D-6146 Alsbach-Hähnlein 2 (DE)**

**EP 0 164 669 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Verfahren zum Überziehen von festen Arzneiformen durch Aufbringen eines Films aus einem flüssigen, filmbildenden Überzugsmittel, dessen flüssiger Bestandteil überwiegend aus Wasser besteht, auf die Arzneiform und Trocknen des Films. Unter Arzneiformen sind hier nicht nur Tabletten, Kapseln und Dragees, sondern insbesondere auch Pellets, Globuli, Granula, Kristalle und ähnliche kompakte oder agglomerierte Arzneistoffpartikel zu verstehen.

Stand der Technik

Wäßrige Überzugsmittel für feste Arzneiformen sind seit langer Zeit allgemein gebräuchlich. Als klassisches Verfahren dieser Gattung ist das Dragieren mit Zuckerlösungen zu nennen, das heute durch Filmdragierverfahren weitgehend abgelöst ist. Bei diesen Verfahren werden neben organischen Überzugsmittellösungen auch wäßrige Überzugsmittel mit wasserlöslichen Cellulosederivaten, Polyäthylenglykolen, Polyvinylalkohol oder Polyvinylpyrrolidon als Bindemittel oder Hilfsbindemittel eingesetzt.

Wäßrige Überzugsmittel haben die Vorteile, daß sie nicht brennbar und ihre Dämpfe nicht explosiv sind und die Verdunstung des Wassers aus dem Überzug keine Umweltbelastung hervorruft. Die wasserlöslichen Bindemittel, die in den bekannten wäßrigen Überzugsmitteln enthalten sind, sind jedoch nicht voll befriedigend. Soweit sie sich von Cellulose ableiten, sind sie bei hoher Luftfeuchtigkeit und Wärme, etwa in den Tropen, dem Angriff von Mikroorganismen ausgesetzt. Die wäßrigen Überzugslösungen sind oft schlecht pigmentierbar und erfordern ein hohes Bindemittel/Pigment-Verhältnis. Beim Trocknen von Überzugsfilmen auf festen Arzneiformen ergeben sie Überzüge von ungenügendem Glanz, unbefriedigender Abriebfestigkeit und Lagerfähigkeit. Zum Teil sind sie hygroskopisch und neigen bei hoher Luftfeuchtigkeit zum Verkleben.

Ein bewähtes Überzugsmittel auf nicht-wäßriger Basis sind alkoholische Lösungen von Acryl- bzw. Methacrylpolymeren, die Seitengruppen mit tertiären Aminogruppen tragen. (DE—PS 1 090 381). Diese Überzugsmittel ergeben auf festen Arzneiformen glatte, glänzende Überzüge, die nicht hygroskopisch und nicht anfällig gegen Mikroorganismen sind. Im sauren Milieu des Magensaftes werden die tert. Aminogruppen des Polymerisats in die Salzform übergeführt. In dieser Form ist das Polymerisat leicht wasserlöslich, so daß sich der Überzug im Magen in wenigen Minuten auflöst. Der einzige Nachteil dieses sehr bewährten Überzugsmittels ist sein Gehalt an brennbaren Lösungsmitteln, die aus Gründen der Sicherheit und der Luftreinhaltung aus der Abluft der Beschichtungsanlage zurückgewonnen werden müssen.

Aus dem US-Patent 3 321 451 sind druckempfindliche Haftkleber bekannt, die zur Befestigung von Verbänden auf die Haut aufgebracht und nach dem Ablösen des Verbands mit Wasser abgewaschen werden können. Die Haftkleber enthalten ein Mischpolymerisat aus einem höheren Acrylsäurealkylester und einem Aminogruppen enthaltenden Comonomeren in der Salzform. Um die für Haftkleber erforderlichen Weichheit zu erreichen, muß der Anteil an höheren Acrylestern so hoch gewählt werden, daß das Copolymerisat in Wasser nicht mehr echt löslich ist, sondern höchstens kolloidal dispergierbar ist. Es wird daher in Form einer alkoholischen Lösung auf die Haut aufgebracht.

Aufgabe und Lösung

Es wurde nach einem in Wasser löslichen Bindemittel zur Bereitung wäßriger Überzugsmittel gesucht, das den bekannten Bindemitteln überlegen ist. Die wäßrige Lösung soll hoch pigmentierbar sein und bei hoher Pigmentierung einen guten Glanz ergeben. Die Überzüge auf festen Arzneiformen sollen abriebfest, wenig spröde, wenig hygroskopisch und mikrobiologisch nicht angreifbar sein. Sie sollen frei von einem unangenehmen Geschmack und Geruch sein und dürfen natürlich nicht toxisch sein.

Es wurde gefunden, daß wasserlösliche Polymerisate, die überwiegend aus Acryl- und/oder Methacrylmonomeren aufgebaut sind und wenigstens 10 Mol-% an Monomereinheiten mit Seitengruppen mit einer tertiären Ammoniumsalzgruppe, deren Stickstoffatom kovalent an die Seitengruppe gebunden ist, enthalten, den bekannten wasserlöslichen Bindemitteln in den vorerwähnten Eigenschaften überlegen sind. Sie werden daher erfindungsgemäß in gelöster Form in flüssigen, filmbildenden Überzugsmitteln, deren flüssiger Bestandteil zu wenigstens 80 Gew.-% aus Wasser besteht, zum Überziehen von festen Arzneiformen eingesetzt.

Zusammensetzung des Überzugsmittels

Als Überzugsmittel wird hier das zum Überziehen von festen Arzneiformen zu verwendende flüssige, filmbildende Mittel bezeichnet, aus dem beim Trocknen des Films ein fester Überzug auf einer Arzneiform entsteht.

Das Überzugsmittel besteht im einfachsten Falle nur aus einem flüssigen Bestandteil und einem darin gelösten Bindemittel, enthält aber meistens weiterhin Pigmente, Füllstoffe und gegebenenfalls Weichmacher und andere Hilfsstoffe, wie Geschmackszusätze, Wirkstoffe, Glanz- und Gleitmittel. Eine besondere Klasse dieser Überzugsmittel enthält neben dem in dem flüssigen Bestandteil gelösten Bindemittel noch ein darin unlösliches, dispergiertes, filmbildendes Bindemittel.

Der flüssige Bestandteil besteht überwiegend, d.h. zu mehr als 80%, vorzugsweise zu 90 bis 100% aus

Wasser. Daneben können wassermischbare flüssige Zusätze mitverwendet werden, die als Lösungsvermittler, Weichmacher oder Filmbildungshilfsmittel wirksam sein können, die unter den üblichen Trocknungsbedingungen schwer- oder nichtflüchtig sind, wie Äthylenglykol, Äthylenglykolmonomethyläther, Propylenglykol, Diäthylenglykol, Polyäthylenglykol, Glycerin, Glycerintriester oder Zitronensäureester. In der Regel enthalten Überzugsmittel für Arzneiformen erhebliche Menge an Pigmenten und/oder Füllstoffen. Das erfindungsgemäß eingesetzte Bindemittel zeichnet sich durch eine gute Pigmentbindefähigkeit aus. Auf 1 Gew.-Teil des Bindemittels können bis zu 3 Gew.-Teile Pigment verarbeitet werden; üblicherweise werden 1 bis 2 Gew.-Teile Pigment verwendet. Es werden die in Überzugsmitteln für Arzneiformen allgemein gebräuchlichen Pigmente und Füllstoffe eingesetzt.

Das Überzugsmittel muß filmbildend sein, d.h. es muß, wenn es auf die Oberfläche einer Arzneiform aufgetragen wird, dort einen geschlossenen Film von 5 bis 50 µm Dicke bilden können, der Beim Trocknen in einen geschlossenen, gleichmäßigen Überzug übergeht. Die Viskosität des Überzugsmittels liegt bei Raumtemperatur im Bereich von 20 bis 50 m Pa s (gemessen durch Rotationsviskosimeter).

Typische Überzugsmittel gemäß der Erfindung enthalten 60 bis 90 Gew.-% des flüssigen Bestandteils, 5 bis 15 Gew.-% des darin löslichen Bindemittels sowie gegebenenfalls bis zu 30 Gew.-% eines ungelösten, dispergierten, filmbildenden Bindemittels und bis zu 25 Gew.-% an Pigmenten und Füllstoffen.

Das gelöste Bindemittel

ist ein überwiegend aus Acryl- und/oder Methacrylmonomeren aufgebautes Polymerisat. Diese Monomeren enthalten eine Gruppe $CH_2{=}CH{-}CO{-}$ bzw. $CH_2{=}C(CH_3){-}CO{-}$ und bilden wenigstens 50, vorzugsweise 80 bis 100 Gew.-% des Polymerisats. Als Comonomere, die nicht der Gruppe der Acryl- und Methacrylmonomeren zugehören, sind Vinylester, wie Vinylacetat, -propionat oder -butyrat, Vinylpyrrolidon, Malein- und Fumar- und Itakonsäure, deren Halb- und Vollester, Styrol, Vinyltoluol und Vinyläther zu nennen.

Charakteristisch für das Polymerisat sind Seitengruppen mit einer tertiären Ammoniumsalzgruppe, die formal durch Salzbildung einer tertiären Aminogruppe mit einem Säureäquivalent entstanden sind. Das Amino-Stickstoffatom ist mit der Seitengruppe kovalent verbunden und auf diese Weise Bestandteil des Polymermoleküls selbst. Der tertiäre Stickstoff ist mit einer Valenz an eine vorzugsweise aliphatische Zwischengruppe gebunden, die mit der Hauptkette des Polymermoleküls verknüpft ist. Die beiden anderen Valenzen tragen aliphatische Reste, vorzugsweise niedere Alkylreste mit 1 bis 4 C-Atomen.

Vorzugsweise gehören die tertiären Aminogruppen zu Monomereinheiten des Polymerisats, die (formal) aus Acryl- oder Methacrylmonomeren der Struktur

$$CH_2{=}\overset{\displaystyle R}{\overset{|}{C}}{-}CO{-}A{-}B{-}N^{+}HR^{1}R^{2},\ X^{-}$$

worin

R ein Wasserstoffatom oder eine Methylgruppe

A eine Sauerstoffatom- oder eine —NH-Gruppe,

B ein aliphatischer oder araliphatischer Kohlenwasserstoffrest mit 2 bis 8 C-Atomen

$R^1$ und $R^2$ gleiche oder verschiedene Alkylreste mit 1 bis 6 C-Atomen oder Cycloalkylreste oder zusammengenommen eine gegebenenfalls durch ein Sauerstoffatom oder eine NH-Gruppe unterbrochene Alkylenkette und

$X^-$ ein einwertiges Säureanion oder ein Äquivalent eines mehrwertigen Säureanions

hervorgegangen sind. Ester (mit A=Sauerstoff) sind bevorzugt. B ist vorzugsweise eine gegebenenfalls verzweigte Alkylengruppe mit wenigstens drei in gerader Kette zwischen dem Ammoniumstickstoffatom und der Gruppe A angeordneten Kohlenstoffatomen. Beispiele für Monomer, die durch Salzbildung in Monomere obiger Struktur übergehen, sind Dimethylaminoäthylacrylat und -methacrylat, 2-Dimethylaminopropyl-acrylat und -methacrylat, Piperidinoäthyl-acrylat und -methacrylat, Morpholinoäthyl-acrylat und -methacrylat, Dimethylamino-benzylacrylat und -methacrylat, 3-Dimethylamino-2,2-dimethyl-propyl-acrylat und -methacrylat, 3-Diäthylamino-2,2-dimethylpropyl-acrylat und -methacrylat, N-Dimethylaminopropyl-acrylamid und -methacrylamid, N-[3-(Dimethylamino)-2,2-dimethylpropyl]-acrylamid und -methacrylamid und N-(3-Morpholinopropyl)-acrylamid und -methacrylamid.

Mit anorganischen oder organischen Säuren gehen die genannten Monomeren, die eine tertiäre Aminogruppe enthalten, an die entsprechenden tertiären Ammoniumsalz über. Geeignet sind Mineralsäuren, wie Salzsäure, Schwefelsäure oder Phosphorsäure sowie saure Salze mehrbasischer Säuren, wie Natriumhydrogensulfat oder Natriumdihydrogenphosphat. Beispiele geeigneter organischer Säuren sind Essigsäure und Citronensäure. Die Säuren müssen physiologisch verträglich sein, da mit ihrer Freisetzung im sauren Milieu des Magens gerechnet werden muß. Die Menge der Säure muß nicht stöchiometrisch der Menge der tertiären Aminogruppen entsprechen; ein Teil dieser Gruppen kann in der Basenform liegen. Auch kann ein Überschuß an Säure eingesetzt werden. Auf diese Weise kann ein pH-Wert eingestellt werden, bei dem die Hilfsstoffe wie Pigmente, Weichmacher u.a. eine optimale Stabilität aufweisen und auch die bestmögliche Verträglichkeit des Überzugsmittels in dem Arzneimittel zu erwarten ist.

Der Gehalt an tertiären Ammoniumsalzgruppen in dem Polymerisat muß ausreichend sein, um es in dem flüssigen Bestandteil des Überzugsmittels löslich zu machen, jedoch wird seine Löslichkeit nicht von diesen Gruppen allein bestimmt. Enthält das Polymerisat beträchtliche Anteile von stark hydrophilen Monomereinheiten, z.B. Hydroxyalkylestern der Acryl- oder Methacrylsäure, so tragen diese selbst zur Löslichkeit in Wasser oder wäßrigen Lösungsmittelgemischen bei. Je größer der Anteil organischer Lösungsmittel an dem flüssigen Bestandteil ist, um so geringer kann der Gehalt an tertiären Ammoniumgruppen und anderen hydrophilen Einheiten sein, um Löslichkeit zu erreichen. Wasserlöslichkeit wird, vor allem wenn keine Carbonamid- oder Hydroxylgruppen vorhanden sind, bei einem Gehalt von wenigstens 10 Mol%, vorzugsweise 15 bis 60 Mol% an Monomereinheiten mit tertiären Ammoniumsalzgruppen, wie den oben formelmäßig dargestellten Acryl- und/oder Methacrylmonomeren, erreicht. Wenn mehr als 20 Mol% an Monomereinheiten mit einem tertiären Amino- bzw. Ammonium-Stickstoffatom in der Seitenkette vorhanden sind, so genügt es, wenn ein Teil davon, beispielsweise mehr als 10 Mol%, in der Ammoniumsalzform vorliegt.

Weitere Acryl- oder Methacrylmonomere, die in der Regel am Aufbau des Polymerisats beteiligt sind, sind die Alkylester der Acryl- und/oder Methacrylsäure, insbesondere die niederen Alkylester mit 1 bis 4 C-Atomen im Alkylrest, sowie Acryl- und Methacrylamid und die bereits erwähnten Hydroxyalkylester, die in der Regel 2 bis 4 C-Atome im Hydroxyalkylrest enthalten. Die Alkylester sind bevorzugt. Unter diesen sind wiederum Methyl-, Äthyl- und n-Butylacrylat besonders bevorzugt.

Die Hydrophilie des Bindemittels soll möglichst nicht höher sein als es für die Löslichkeit in dem flüssigen Bestandteil erforderlich ist. Werden dem Polymerisat bei seiner Herstellung mehr hydrophile Anteile als zur Löslichkeit erforderlich einverleibt, so kann der damit erzeugte Überzug hygroskopisch sein und bei hoher Luftfeuchtigkeit klebrig werden. Es gehört zu den Vorzügen der Erfindung, daß eine solche Hydroskopizität bei einer für die Löslichkeit gerade ausreichenden Hydrophilie noch nicht erreicht wird.

Neben der Hydrophilie ist die Härte des Bindemittels von Bedeutung. Damit der Überzug die erwünschte Elastizität und Abriebfestigkeit erreicht, darf das Bindemittel nicht zu hart sein. Erwünscht ist eine Zugfestigkeit $\delta_R > 10$ N/mm$^2$ und eine Reißdehnung $\varepsilon_R > 250\%$ (gemessen nach DIN 53 455). Die Monomereinheiten mit tertiären Amino-Seitengruppen ergeben, wenn sie allein das Polymerisat aufbauen, meist verhältnismäßig harte Bindemittel. Durch einen ausreichenden Anteil weicher Comonomerer, vor allem niederer Ester der Acrylsäure, läßt sich eine geringere Härte einstellen. Zu dem gleichen Zweck kann das Bindemittel äußere Weichmacher enthalten.

Das Polymerisat kann durch radikalische Polymerisation seiner Monomerbestandteile in wäßriger oder wäßrig-alkoholischer Lösung erzeugt werden. Es wird jedoch bevorzugt, zunächst ein nicht neutralisiertes Polymerisat herzustellen, indem man anstelle der Monomeren mit einer tertiären Ammoniumsalzgruppe das zugrundeliegende Monomere in der Basenform einsetzt. Für die radikalische Polymerisation dieser Monomerengemische stehen verschiedene, seit langem bekannte Polymerisationsverfahren zur Verfügung, z.B. die Polymerisation in organischen Lösungsmitteln, die Polymerisation in Substanz und, da die Polymerisate in der Basenform weniger wasserlöslich sind, in günstigen Fällen auch die Emulsionspolymerisation in wäßriger Phase. Die organischen Polymerisatlösungen und wäßrigen Polymerisatdispersionen lassen sich z.B. durch Sprühtrocknung in Pulverprodukte überführen. Die Substanzpolymerisate werden im Extruder aufgeschmolzen und zu einem feinen Granulat extrudiert.

Das Molekulargewicht des Polymerisats beeinflußt die Viskosität des Überzugsmittels in Abhängigkeit von seiner Konzentration, bezogen auf den flüssigen Bestandteil. Es liegt vorzugsweise im Bereich zwischen 50.000 und 250.000. Die Viskosität der Lösung des Polymerisats in dem flüssigen Bestandteil soll—in Abwesenheit von Pigmenten und Füllstoffen—nicht mehr als 100 m Pa s, vorzugsweise etwa 20 bis 50 m Pa s betragen, wobei der Polymerisatgehalt mindestens 5 Gew.-% dieser Lösung ausmacht.

Die Herstellung des Überzugsmittels

Die Elastizität und Härte des Arzneimittel-Überzugs hängen von dem Pigment-Bindemittel-Verhältnis und von der Härte des Bindemittels selbst ab. Daher muß bei der Wahl des Bindemittels die vorgesehene Pigment-Konzentration entsprechend berücksichtigt werden.

In der Praxis ist es vorteilhaft, das flüssige Überzugsmittel aus einem in der Basenform vorliegenden Polymerisat zu erzeugen. Dieses wird in Form eines mittelfeinen bis feinen Pulvers oder in Form von Granulat unter Rühren und Zusatz einer Säure in dem flüssigen Bestandteil gelöst. Vorzugsweise wird kaltes Wasser verwendet. Es ist besonders zweckmäßig, solche Polymerisatpulver einzusetzen, die sich im nicht neutralisierten Zustand zur Herstellung von Arzneimittelüberzügen aus organischen Lösungen eignen; solche Überzugslösungen sind seit Jahrzehnten bekannt.

Die Härte des Bindemittels wird durch die Salzbindung im Vergleich zu dem zugrundeliegenden basischen Polymerisat erhöht.

So wurden, ausgehend von einem Polymerisat in der Basenform, durch Neutralisation mit Salzsäure, Citronensäure und Phosphorsäure und Zusatz von 20% Weichmacher Bindemittel erhalten, die sich in der

Lage des Dämpfungsmaximums im Torsionsschwingungsversuch ($T_{1\ max}$ nach DIN 53 445), welches ein Maß für die Bindemittelhärte darstellt, folgendermaßen unterschieden

| | |
|---|---|
| Chlorid-Form | $T_{1max}$=86°C |
| Citrat-Form | $T_{1max}$=59°C |
| Phosphat-Form | $T_{1max}$=54°C |

Durch Zusatz von beispielsweise 10 bis 20 Gew.-% (bezogen auf das Polymerisatgewicht) eines Weichmachers läßt sich die Härte des Bindemittels auf einen anwendungstechnisch günstigen Wert erniedrigen. Als Weichmacher eignen sich flüssige, wenigstens begrenzt wasserlösliche organische Stoffe, die mit dem Polymerisat verträglich sind unter den Bedingungen des überzugsverfahrens nicht oder nicht in einem wesentlichen Maße flüchtig sind. Verträglichkeit ist gegeben, wenn sich aus dem unpigmentierten Bindemittel und dem Weichmacher beim Trocknen ein klarer Film herstellen läßt oder wenn sich das Bindemittel in einer größeren Menge des Weichmachers löst. Als Weichmacher geeignete Stoffe haben in der Regel ein Molekulargewicht zwischen 200 und 20.000 und enthalten eine oder mehrere hydrophile Gruppen im Molekül, z.B. Hydroxyl-, Äther- oder Aminogruppen. Beispiele geeigneter Weichmacher sind Citronensäurealkylester, Glycerintriacetat und Polyäthylenglykole 500 bis 20.000.

Neben dem Ammonium-Seitengruppen enthaltenden Polymerisat können andere gelöst Bindemittel in den neuen Überzugsmassen mitverwendet werden, gegebenenfalls auch solche, die sich nicht als alleiniges Bindemittel eignen würden. Als Beispiele seien Polyvinylalkohol, Polyvinylpyrrolidon und Polyäthylenglykole genannt.

Von besonderer Bedeutung ist die Anwendung der Polymerisate zusammen mit einem filmbildenden, aber nicht gelösten, sondern in wäßriger Phase dispergierten Bindemittel. Es liegt in einer Teilchengröße zwischen 0,05 und 1 µm vor und bildet den überwiegenden Teil des gesamten Bindemittelgehalts des Überzugsmittels. Die dispergierten Teilchen müssen unter den Verfahrensbedingungen zu einem zusammenhängenden Film zusammenfließen. Aus der DE—PS 16 17 351 (GB—PS 1 213 348) ist es bekannt, Arzneimittelüberzüge aus derartigen filmbildenden wäßrigen Dispersionen von Bindemitteln, die im gesamten physiologischen pH-Bereich unlöslich sind, herzustellen. Damit der Wirkstoff aus der unlöslichen Umhüllung freigesetzt werden kann, wird in diese eine wasser- oder alkalilösliche Substanz eingearbeitet, die im Magen-Darm-Trakt herausgelöst wird. Durch die entstehenden Poren wird der Wirkstoff freigesetzt. Die dabei als wasserlösliche Substanzen eingesetzten Polymeren, wie Polyäthylenglykole, Polyvinylpyrrolidone, Polyvinylalkohole oder Stärke, haben den Nachteil, daß sie bis zu beträchtlichen Zusatzmengen fast keine permeabilitätssteigernde Wirkung entfalten, aber bei weiterer Steigerung der Zusatzmenge plötzlich durch Zerstörung des Filmverbands zu einer unerwünscht starken Permeabilitätssteigerung oder Quellung führen, verbunden mit einem Verlust wichtiger Filmeigenschaften, wie Härte, Glanz und Abriebfestigkeit.

Überraschenderweise haben die erfindungsgemäß verwendeten tert. Ammoniumgruppen enthaltenden Polymerisate die Wirkung, daß sie die Permeabilität von Arzneimittelüberzügen aus Dispersionen unlöslicher Bindemittel proportional zu ihrer Zusatzmenge erhöhen. Überzüge aus 30% des erstgenannten und 70% des letztgenannten Bindemittels können die Durchlässigkeit einer dünnen Papiermembran haben, ohne daß die mechanischen Eigenschaften wesentlich beeinträchtigt würden. Auf diese Weise lassen sich Filmüberzüge von ausgezeichneter Härte und Elastizität erzeugen.

Die Herstellung von Arzneimittelüberzügen aus einem flüssigen, filmbildenden Überzugsmittel auf wäßriger Basis, welches als Bindemittel ein Gemisch aus

A) 1 bis 49 Gew.-% des Polymerisats mit tert.-Ammoniumsalz-Seitengruppen und

B) 99 bis 51 Gew.-% eines filmbildenden, im physiologischen pH-Bereich nicht wasserlöslichen, dispergierten Bindemittels,

enthält, stellt eine bevorzugte Ausführungsform der Erfindung dar. Die oben mit B bezeichneten Polymeren sind in den pharmakologisch unbedenklichen filmbildenden wäßrigen Dispersionen enthalten, die schon in der DE—PS 16 17 351 erwähnt sind; beispielsweise Polyvinylester, Polyvinylacetale, Polyvinylchlorid, Butadien-Styrol-Mischpolymere und insbesondere Polyacrylsäureester. Die letzteren sind in der Regel zu mehr als 90 Gew.-% aus (vorzugsweise niederen) Alkylestern der Acryl- oder/und Methacrylsäure aufgebaut.

Das Überzugsverfahren

Nach dem Verfahren der Erfindung können alle festen Arzneiformen überzogen werden, die Tabletten, Dragees, Pillen, Granulate, Pellets, Wirkstoffkristalle oder Kapseln. Auch lassen sich alle gebräuchlichen Überzugsverfahren anwenden, wie das Kesseldragierverfahren oder das Wirbelschicht-Dragierverfahren. Ebenso können die neuen Überzugsmittel zum gleichzeitigen Überziehen und Granulieren von Wirkstoffpulvern verwendet werden, wobei man Granulate erhält, die sich zu Matrixtabletten verpressen oder in Kapseln abfüllen lassen.

Die Mange des Überzugsmittels wird im allgemeinem so berechnet, daß Überzüge von 5 bis 50 µm Dicke auf der Arzneiform entstehen. Diese Überzüge können gewünschtenfalls aus mehreren Schichten, gegebenenfalls auch von unterschiedlicher Zusammensetzung, aufgebaut werden.

Die Formulierung des Überzugsmittels richtet sich nach dem Überzugsverfahren und den

gewünschten Filmeigenschaften. Es lassen sich z.B. niedrigviskose Suspensionen mit einem Feststoffgehalt bis zu 20% und einer Viskosität unter 100 m Pa s für Sprühverfahren einsetzen. Solche Suspensionen können z.B. in Wirbelschichtanlagen eingesprüht oder auf Arzneiformen in einem rotierenden Kessel aufgesprüht werden. Überzugsmittel von höherer Viskosität und höherem Feststoffgehalt lassen sich nach dem Vorbild des klassischen Kessel-Dragierverfahrens in Einzelportionen auf Arzneiformen aufgießer.

Während des Filmbildungsvorganges wird im allgemeinen mit Warmluft von 40 bis 80°C getrocknet. Bei der bevorzugten Ausführungsform, bei der das flüssige Bestandteil allein aus Wasser besteht, kann die Abluft unmittelbar und ohne Umweltbelastung in die Atmosphäre entlassen werden. Nachdem die gewünschte Menge des Überzugsmittels aufgebracht ist, empfiehlt sich eine trocknungszeit von einigen Stunden im Warmluftschrank oder in einem Warmluftstrom bei Temperaturen von 40 bis 60°C. Man erhält bei zweckmäßiger Formulierung Arzneiformen mit gefälligem Aussehen, hohem Glanz, hoher Abriebfestigkeit und guter Lagerungsfähigkeit selbst im tropischen Klima.

Die Freisetzungseigenschaften der überzogenen Arzneiformen

Werden die Ammoniumsalzgruppen enthaltenden Polymerisate als alleinige Bindemittel oder im Gemisch mit anderen wasserlöslichen Bindemitteln angewendet, so erhält man Arzneiformen mit einem leicht wasserlöslichen Überzugsfilm, der sich im physiologischen pH-Bereich von etwa 1 bis 8 des Verdauungstraktes innerhalb weniger Minuten auflöst und den enthaltenen Wirkstoff sofort freigibt. Diese Auflösung des Films ist im Magen unabhängig von der Nahrungsaufnahme, pathologischen Zuständen und tritt ebenso schnell bei operativ verkleinertem oder entferntem Magen ein. Selbst wenn die Arzneiform sehr schnell in den Darmbereich eintritt, löst sie sich dort bei einem pH-Wert von 5 bis 8 schnell auf. Andererseits ist die Lösungsgeschwindigkeit auch langsam genug, um eine Geschmacksüberdeckung beim Schlucken zu erreichen. Jedoch wird der Überzug auch beim längeren Lutschen im Mund abgelöst, so daß sich der Überzug auch für Sublingualtabletten eignet.

Bei der Anwendung in Kombination mit nicht wasserlöslichen wäßrigen Überzugsmitteldispersionen lassen sich alle Übergänge zwischen sofort zerfallenden bis zu echten Retard-Präparaten erzeugen, wobei die Freisetzungsgeschwindigkeit vom pH-Wert des umgebenden Milieus praktisch unabhängig ist. Vor allem die Kombinationen von wäßrigen Dispersionen neutraler Polyacryl- und Polymethacrylester-Polymerisate mit den erfindungsgemäß eingesetzten Polymerisaten von Acryl- und Methacrylmonomeren zeichnen sich durch eine gute Verträglichkeit aus. Daher kann man aus derartigen Kombinationen ein variables, an die Permeationseigenschaften und therapeutischen Anforderungen der verschiedenen Wirkstoffe gut anpaßbares System von Überzugsrezepturen mit abgestufter Permeabilität aufbauen.

Beispiel 1

Aus 50 Gewichtsteilen Dimethylaminoäthylmethacrylat, 25 Teilen Methylmethacrylat und 25 Teilen n-Butylmethacrylat wird durch radikalische Polymerisation ein Substanzpolymerisat hergestellt. bei 140°C unter Entgasen extrudiert, das Gleichkorngranulat gemahlen und Grobanteile über 0,25 mm abgesiebt.

1600 g des Polymerpulvers (enthaltend 5,1 Mol Aminogruppen) wurden in 8,6 kg Wasser suspendiert und 540 g=2,6 Mol Citronensäure-1-hydrat ($C_6H_5O_7 \cdot H_2O$ MG 210,14) entsprechend 7,7 Äquivalente Carboxylgruppen eingerührt. Man rührt kräftig weiter, so daß die Polymerpartikel sich nicht zusammenballen oder an den Wandungen festkleben. Nach etwa 2 Std. ist eine schwach trübe, gelbliche Lösung entstanden, die einen pH-Wert von 3,25 zeigt und in einer Schicht von ca. 100 µm ausgegossen. bei Raumtemperatur zu einem klaren, nicht klebenden, spröden Film auftrocknet. 224 g der obigen Lösung mit 20% Trockengehalt an Polymerisat wurden mit einer Lösung von 3,4 g Polyethylenglykol 6000 in 30 g Wasser als Weichmacher und 15 g Talkum als Trennmittel versetzt und in einem Dragierkessel von 35 cm 0 bei einer Umdrehungsgeschwindigkeit von 40 U/min auf 3 kg Tabletten von 7 mm 0, 3,5 mm Höhe und 140 mg Einzelgewicht mit einer Luftdruckspritzpistole mit einer Düse von 1 mm 0 und einem Sprühdruck von $0{,}6 \times 10^5$ Pa (0.6 bar) innerhalb von 45 min aufgesprüht.

Es wurde kontinuierlich während des Sprühens durch Einblasen von Warmluft von etwa 70°C getrocknet. Es entsteht ein glatter. mattglänzender Filmüberzug. der sich sowohl in Wasser als auch in künstlichem Magensaft schnell auflöst, so daß die überzogenen Tabletten in beiden Medien innerhalb von weniger als 2 min zerfallen.

Beispiel 2

224 g der in Beispiel 1 beschriebenen Lösung des Polymerisates in Citronensäure mit 20% Trockengehalt an Polymersalz wurden mit einer Lösung von 3,4 g Polyethylenglykol 6000 in 30 g Wasser und 200 g einer 30 %igen Pigmentsuspension folgender Zusammensetzung vermischt:

| | |
|---|---|
| Talkum | 32 g |
| Titandioxid | 12 g |
| Rotlack | 12 g |
| Polyethylenglykol 6000 | 4 g |
| Wasser | 140 g |
| | 200 g |

Der Auftrag der Mischung erfolgt ebenfalls auf 3 kg Tabletten der in Beispiel 1 angegebenen Größe unter den dort genannten Sprühbedingungen, wobei die gesamte Sprühzeit jedoch 75 min betrug. Es entstanden gleichmäßig gefärbte, mattglänzende Filmdragees, die in Wasser und künstlichem Magensaft gleichermaßen schnell innerhalb von weniger als 2 Minuten zerfielen.

Beispiel 3

100 g eines nach Beispiel 1 hergestellten Substanzpolymerisates aus 50% Dimethylaminoäthylmethacrylat und je 25% Methyl- und Butylmethacrylat einer Korngröße<0,25 mm. entsprechend 0,32 Mol Aminogruppen, wurden in 419 g Wasser suspendiert und unter Rühren 480 g einer wäßrigen 1 m Lösung von Natriumdihydrogenphosphat, entsprehend 0,96 Äquivalent Säure, zugesetzt. Nach einer Rührzeit von 2 Std. ist eine klare, gelbliche Lösung entstanden.

335 g der obigen Lösung mit 17,5% Trockengehalt wurden mit einer Lösung von 3,4 g Polyethylenglykol in 30 g Wasser versetzt und mit 200 g der in Beispiel 2 beschriebenen Pigmentsuspension vermischt.

Beim Aufsprühen auf 3 kg Tabletten analog zu Beispiel 2 entstanden relativ glatte und leicht glänzende Filmdragees, die in Wasser und künstlichem Magensaft innerhalb von 2 min vollständig zerfielen.

Beispiel 4

Aus 50 Gewichtsteilen 3-Dimethylamino-2,2-dimethyl-propyl-1-methacrylat und 50 Gewichtsteilen Methylmethacrylat wurde durch Emulsionspolymerisation in Wasser mit 3% Polyoxyethylen(20)sorbitan-mono-oleat (Tween® 80) und 0,1% Natriumdodecylsulfat als Emulgatoren und einem Peroxid-Initiator eine Kunststoffdispersion hergestellt und daraus durch Sprühtrocknung ein Pulver gewonnen.

50 g dieses Pulvers, enthaltend 0,125 Mol Aminogruppen, wurden in 250 g Wasser suspendiert und unter Rühren 13 g Citronensäure-1-hydrat, entsprechend 0,186 Äquivalenten Carboxylgruppen, zugesetzt. Nach 3 Stunden ist eine leicht gelbliche trübe Lösung entstanden. Nach dem Filtrieren erhält man 310 g einer fast klaren, etwa 20 %igen Lösung, die nach Beispiel 2 weiterverarbeitet werden kann.

Beispiel 5

167,5 g einer Lösung nach Beispiel 3, die 17,5% Trockensubstanz eines mit Natriumdihydrogenphosphat gebildeten Polymersalzes enthielt, wurden mit 112 g eines Emulsionspolymerisates aus neutralen (Meth)acrylsäureestern (70% Ethylacrylat. 30% Methylmethacrylat) mit 30% Trockengehalt vermischt und mit 95 g Wasser verdünnt. Nach Zugabe von 15 g Talkum wurde das Gemisch auf 3 kg Tabletten von 7 mm 0 in einem Dragierkessel innerhalb von 50 min aufgesprüht, wobei die Verfahrensbedingungen nach Beispiel 1 eingehalten wurden. Es wurden keine Verklebungstendenzen während des Auftragsverfahrens beobachtet, was sonst bei alleiniger Anwendung des neutralen Emulsionspolymerisates störend ist. Die Zerfallszeit der fertig überzogenen Tabletten betrug weniger als 2 min.

Beispiel 6

600 g eines extrudierten Copolymerisates aus gleichen Teilen Dimethylaminoäthylmethacrylat und Methylmethacrylat wurden in 3,6 l Wasser eingerührt und 301 g Natriumdihydrogenphosphat-2-hydrat zugegeben. Die Granulatkörner von 1—2 mm lösen sich bei Raumtemperatur innerhalb von 72 Stunden vollständig auf. Die Lösung wurde mit Wasser ad 6000 g verdünnt. 135 g dieser Lösung. die 13.5 g des Copolymerisates (Base) enthielt, wurden mit 405 g eines Emulsionspolymerisates mit 30% Trockengehalt (121.5 g Trockensubstanz) aus gleichen Teilen Methylmethacrylat und Ethylacrylat vermischt. Das Verhältnis des basischen Copolymerisates zu neutralem Copolymerisat betrug somit 1:9.

Es wurden außerdem noch 200 g einer 15 %igen Pigmentsuspension zugefügt, die folgende Zusammensetzung hatte:

| | |
|---|---|
| Talkum | 8 g |
| Mg-stearat | 10 g |
| Titandioxid | 4 g |
| Gelblack E 102 | 5 g |
| Polyäthylenglykol 6000 | 3 g |
| Wasser | 170 g |
| | 200 g |

Diese pigmentierte Lackmischung wurde nun in einem Laborwirbelschichtgerät (Uni-Glatt) auf 1,5 kg Chlorphenaminmaleat-Pellets einer Korngröße von 0,5—1,2 mm und einem Wirkstoffgehalt von 8% aufgesprüht. Dazu wurde Warmluft von 40°C in das Gerät von unten eingeblasen und die von oben in das Wirbelbett hereinragende Sprühdüse mit einer Bohrung von 1 mm 0 mit einer Zerstäuberluft von $10^5$–$2\times10^5$ Pa (1—2 bar) betrieben. Der Sprühauftrag ist nach 65 min abgeschlossen. Die überzogenen Pellets zeigen in künstlichen Verdauungssäften eine zeitlich verzögerte Wirkstoffabgabe, so daß etwa 80% des Wirkstoffes innerhalb von 6 Std. freigesetzt sind.

Beispiel 7

Werden in der Rezeptur nach Beispiel 6 270 g der Lösung des basischen Copolymerisates, die 27 g Trockensubstanz enthält, mit 360 g eines Emulsionspolymerisates mit 30% Trockengehalt (108 g Trockensubstanz) aus gleichen Teilen Methylmethacrylat und Ethylacrylat vermischt, so daß das Verhältnis des basischen Copolymerisates zum neutralen Copolymerisat 2:8 beträgt, mit der obigen Pigmentsuspension vermischt und die Chlorphenaminmaleat-Pellets analog zum Beispiel 6 überzogen, so ist die Wirkstoffabgabe beschleunigt; es werden dann etwa 80% des Wirkstoffes innerhalb von 4 Std. freigesetzt.

**Patentansprüche**

1. Verfahren zum Überziehen von Arzneiformen durch Aufbringen eines Films aus einem flüssigen, filmbildenden Überzugsmittel, dessen flüssiger Bestandteil zu wenigstens 80 Gew.-% aus Wasser besteht, auf die Arzneiform und Trocknen des Films, dadurch gekennzeichnet, daß ein Überzugsmittel eingesetzt wird, welches ein gelöstes Polymerisat enthält, das überwiegend aus Acryl- und/oder Methacrylmonomeren aufgebaut ist und wenigstens 10 Mol-% an Monomereinheiten mit Seitengruppen mit einer tertiären Ammoniumsalzgruppe, deren Stickstoffatom kovalent an die Seitengruppe gebunden ist, enthält.

2. Verfahren nach Anspruch 1, dadurch gekennzeichnet, daß ein Überzugsmittel eingesetzt wird, das als Polymerisat von Acryl- oder/und Methacrylmonomeren ein Homo- oder Copolymerisat von Monomeren der Struktur

$$CH_2{=}\overset{\overset{\large R}{|}}{C}{-}CO{-}A{-}B{-}N^{+}HR^{1}R^{2},\ X^{-}$$

worin

R ein Wasserstoffatom oder eine Methylgruppe

A ein Sauerstoffatom oder eine —NH-Gruppe,

B ein aliphatischer oder araliphatischer Kohlenwasserstoffrest mit 2 bis 8 C-Atomen,

$R^1$ und $R^2$ gleiche oder verschiedene Alkylreste mit 1 bis 6 C-Atomen oder Cycloalkylreste oder zusammengenommen eine gegebenenfalls durch ein Sauerstoffatom oder eine NH-Gruppe unterbrochene Alkylenkette und

$X^-$ ein einwertiges Säureanion oder ein Äquivalent eines mehrwertigen Säureanions ist,

enthält.

3. Verfahren nach Anspruch 2, dadurch gekennzeichnet, daß ein Überzugsmittel eingesetzt wird, worin das genannte Polymerisat Einheiten eines niederen Alkylesters der Acryl- und/oder Methacrylsäure enthält.

4. Verfahren nach den Ansprüchen 1 bis 3, dadurch gekennzeichnet, daß ein Überzugsmittel eingesetzt wird, worin das genannte Polymerisat zu 10 bis 60 Mol% aus Monomereinheiten mit einem tertiären Ammonium-Stickstoffatom und zu 40 bis 90 Mol% aus Monomereinheiten von niederen Alkylestern der Acryl- oder/und Methacrylsäure zusammengesetzt ist.

5. Verfahren nach den Ansprüchen 1 bis 4, dadurch gekennzeichnet, daß ein Überzugsmittel eingesetzt wird, worin das genannte Polymerisat zusätzlich Monomereinheiten mit einem nicht durch Säuren neutralisierten tert.Amino-Stickstoffatom enthält.

6. Verfahren nach den Ansprüchen 1 bis 5, dadurch gekennzeichnet, daß ein Überzugsmittel eingesetzt wird, das einen wenigstens begrenzt wasserlöslichen, mit dem Polymerisat verträglichen Weichmacher enthält.

7. Verfahren nach den Ansprüchen 1 bis 6, dadurch gekennzeichnet, daß ein Überzugsmittel eingesetzt wird, das zusätzlich ein in dem Überzugsmittel unlösliches, dispergiertes Bindemittel enthält, welches den überwiegenden Teil des genannten Bindemittelgehaltes des Überzugsmittels ausmacht.

8. Verfahren nach Anspruch 7, dadurch gekennzeichnet, daß ein Überzugsmittel eingesetzt wird, das

A) 1 bis 49 Gew.-% des Polymerisats mit tert. Ammoniumsalz-Seitengruppen,

B) 99 bis 51 Gew.-% des filmbildenden, im physiologischen Bereich nicht wasserlöslichen dispergierten Bindemittels

enthält.

9. Verfahren nach den Ansprüchen 7 oder 8, dadurch gekennzeichnet, daß ein Überzugsmittel eingesetzt wird, worin das dispergierte Bindemittel ein Polyacrylester ist, der zu mehr als 90 Gew.-% aus Alkylestern der Acryl- oder/und Methacrylsäure aufgebaut ist.

10. Verfahren nach den Ansprüchen 1 bis 9, dadurch gekennzeichnet, daß das Überzugsmittel auf die zu überziehenden Arzneiformen aufgesprüht wird.

**Revendications**

1. Procédé d'enduction de formes médicamenteuses par dépôt et séchage sur celles-ci d'un film d'un matériau d'enduction filmogène liquide dont le composant liquide est constitué d'au moins 80% en poids d'eau, caractérisé en ce qu'on utilise un agent filmogène qui contient un polymère en solution constitué

essentiellement de monomères acryliques et/ou méthacryliques et contenant au moins 10 mol, % d'unités monomères à groupes latéraux comprenant un groupe sel d'ammonium tertiaire dont l'atome d'azote est fixé par liaison covalente au groupe latéral.

2. Procédé selon la revendication 1, caractérisé en ce qu'on utilise un matériau d'enduction qui, en tant que polymère de monomères acryliques et/ou méthacryliques, contient un homo- ou copolymère de monomères de structure

$$CH_2{=}\overset{\displaystyle R}{\overset{|}{C}}{-}CO{-}A{-}B{-}N^+HR^1R^2,\ X^-$$

dans laquelle

R est un atome d'hydrogène ou un groupe méthyle

A un atome d'oxygène ou un groupe —NH—

B un radical d'hydrocarbure aliphatique ou araliphatique avec 2 à 8 atomes de C

$R^1$ et $R^2$ des radicaux alkyle identiques ou différents avec 1 à 6 atomes de C ou des radicaux cycloalkyle ou, pris ensemble, une chaîne alkylène éventuellement interrompue par un atome d'oxygène ou un groupe—NH— et

$X^-$ un anion acide monovalent ou un équivalent d'un anion acide multivalent.

3. Procédé selon la revendication 2, caractérisé en ce qu'on utilise un matériau d'enduction dans lequel le polymère mentionné contient des unités d'un ester d'alkyle de l'acide acrylique et/ou méthacrylique à faible poids moléculaire.

4. Procédé selon les revendications 1 à 3, caractérisé en ce qu'on utilise un matériau d'enduction dans lequel le polymère mentionné est composé de 10 à 60 mol. % d'unités monomères avec un atome d'azote sous forme ammonium tertiaire et de 40 à 90 mol. % d'unités monomères d'esters d'alkyle de l'acide acrylique et/ou méthacrylique à faible poids moléculaire.

5. Procédé selon les revendications 1 à 4, caractérisé en ce qu'on utilise un matériau d'enduction dans lequel le polymère mentionné contient en outre des unités monomères comprenant un atome d'azote sous forme d'amino tertiaire non neutralisé par des acides.

6. Procédé selon les revendications 1 à 5, caractérisé en ce qu'on utilise un matériau d'enduction qui contient un plastifiant compatible avec le polymère et au moins partiellement hydrosoluble.

7. Procédé selon les revendications 1 à 6, caractérisé en ce qu'on utilise un matériau d'enduction qui contient en outre un liant dispersé dansle matériau d'enduction et insoluble dans celui-ci et qui forme la plus grande part de la teneur en liant du matériau d'enduction.

8. Procédé selon la revendication 7, caractérisé en ce qu'on utilise un matériau d'enduction qui contient

A) 1 à 49% en poids du polymère à groupes latéraux de sels d'ammonium tertiaires,

B) 99 à 51% en poids du liant filmogène en dispersion non hydrosoluble dans le domaine physiologique.

9. Procédé selon les revendications 7 ou 8, caractérisé en ce qu'on utilise un matériau d'enduction dans lequel le liant en dispersion est un ester polyacrylique constitué à plus de 90% en poids d'esters d'alkyle de l'acide acrylique et/ou méthacrylique.

10. Procédé selon les revendications 1 à 9, caractérisé en ce que le matériau d'enduction est vaporisé sur les formes à enduire.

**Claims**

1. Process for coating pharmaceutical preparations by applying a film consisting of a liquid, film-forming coating agent the liquid component of which is at least 80 wt% water, to the pharmaceutical preparation and drying the film, characterised in that a coating agent is used which contains a dissolved polymer which is synthesised predominantly from acrylic and/or methacrylic monomers and contains at least 10 mol-% of monomer units having side groups with a tertiary ammonium salt group the nitrogen atom of which is covalently bonded to the side group.

2. Process according to claim 1, characterised in that a coating agent is used which contains as the polymer of acrylic and/or methacrylic monomers a homo- or copolymer of monomers of the structure

$$CH_2{=}\overset{\displaystyle R}{\overset{|}{C}}{-}CO{-}A{-}B{-}N^+HR^1R^2,\ X^-$$

wherein

R is a hydrogen atom or a methyl group,

A is an oxygen atom or an —NH-group,

B is an aliphatic or araliphatic hydrocarbon radical having 2 to 8 carbon atoms,

$R^1$ and $R^2$ are identical or different alkyl groups having 1 to 6 carbon atoms or cycloalkyl groups or together represent an alkylene chain optionally interrupted by an oxygen atom or an —NH-group and

$X^-$ is a monovalent acid anion or an equivalent of a monovalent acid anion.

3. Process according to claim 2, characterised in that a coating agent is used wherein said polymer

contains units of a lower alkyl ester of acrylic and/or methacrylic acid.

4. Process according to claims 1 to 3, characterised in that a coating agent is used wherein said polymer is made up of 10 to 60 mol-% of monomer units having a tertiary ammonium-nitrogen atom and 40 to 90 mol-% of monomer units of lower alkyl esters of acrylic and/or methacrylic acid.

5. Process according to claims 1 to 4, characterised in that a coating agent is used wherein the said polymer additionally contains monomer units having a tert.-amino-nitrogen atom which is not neutralised by acids.

6. Process according to claims 1 to 5, characterised in that a coating composition is used which contains a plasticiser which is at least partially water-soluble and is compatible with the polymer.

7. Process according to claims 1 to 6, characterised in that a coating agent is used which additionally contains a binder which is dispersed but insoluble in the coating agent and which makes up the majority of the binder content of the coating agent.

8. Process according to claim 7, characterised in that a coating agent is used which contains

A) 1 to 49% by weight of the polymer with tert.-ammonium salt side groups,

B) 99 to 51% by weight of the film-forming dispersed binder which is water-insoluble in the physiological range.

9. Process according to claims 7 or 8, characterised in that a coating agent is used wherein the dispersed binder is a polyacrylic ester made up of more than 90% by weight of alkyl esters of acrylic and/or methacrylic acid.

10. Process according to claims 1 to 9, characterised in that the coating agent is sprayed onto the pharmaceutical preparations which are to be coated.